# EUROPEAN PATENT SPECIFICATION

(11) **EP 2 844 634 B1**
(45) Date of publication and mention of the grant of the patent: **14.04.2021**
(21) Application number: 13784429.6
(22) Date of filing: 24.04.2013
(51) Int. Cl.: C07C 67/00, C07C 67/31, C07C 67/313, C07C 69/716, C07C 309/73, C07D 263/34

(54) **PROCESS FOR THE PREPARATION OF INTERMEDIATE COMPOUNDS FOR PREPARING VITAMIN B6**
VERFAHREN ZUR HERSTELLUNG VON ZWISCHENVERBINDUNGEN ZUR HERSTELLUNG VON VITAMIN B6
PROCÉDÉ POUR LA PRÉPARATION DES COMPOSÉS INTERMÉDIAIRES POUR LA PRÉPARATION DE VITAMINE B6

(30) Priority: 03.05.2012 CN 201210135467
(43) Date of publication of application: 11.03.2015
(73) Proprietor: DSM IP Assets B.V., 6411 TE Heerlen (NL)
(72) Inventor: YANG, Kai, Shangai 201203 (CN); HUANG, Jing, Shangai 201203 (CN); EISELE, Frank, 4303 Kaiseraugst (CH)
(74) Representative: Kurt, Manfred
(86) International application number: PCT/CN2013/000467
(87) International publication number: WO 2013/163889

(56) References cited:
- EP-A1- 2 096 111
- EP-B1- 1 404 324
- WO-A1-2011/108696
- WO-A1-2011/108696
- WO-A1-2012/109100
- BE-A1- 893 835
- US-A- 2 376 033
- US-A- 2 503 699
- US-A- 5 468 761
- BIOCHEM. J., vol. 32, no. 6, 1938, pages 1033-1053, XP002744158,
- TOSHIYASU ISHIMARU ET AL: "[alpha]-HALO-[beta]-DICARBONYL COMPOUNDS. NOVEL CARBOXYL PROTECTING REAGENTS", CHEMISTRY LETTERS, no. 11, 1977, pages 1313-1316, XP55211413, ISSN: 0366-7022, DOI: 10.1246/cl.1977.1313
- DATABASE CAPLUS [Online] CHEMICAL ABSTRACTS SERVICE, COLUMBUS, OHIO, US; OKAZAKI, TOSHIO ET AL: "Preparation of N-[(substituted five-membered heteroaryl)carbonyl]guanidine derivatives as Na+/H+ exchanger inhibitors", XP002744159, retrieved from STN Database accession no. 1998:424227 & WO 98/27061 A1 (YAMANOUCHI PHARMACEUTICAL CO., LTD., JAPAN; MERCK PATENT G.M.B.H.) 25 June 1998 (1998-06-25)
- DATABASE CAPLUS [Online] CHEMICAL ABSTRACTS SERVICE, COLUMBUS, OHIO, US; BOYD, G. V.: "Product class 12 : oxazoles", XP002744160, retrieved from STN Database accession no. 2002:835619 & BOYD, G. V.: "Product class 12 : oxazoles", SCIENCE OF SYNTHESIS , 11, 383-479 CODEN: SSCYJ9, 2002,
- DATABASE CAPLUS [Online] CHEMICAL ABSTRACTS SERVICE, COLUMBUS, OHIO, US; JAKOBSEN, HANS JOERGEN ET AL: "Enamine chemistry. V . Introduction of the benzoyloxy group into some .beta.-oxoesters and .beta.-diketones. A new route to oxazoles", XP002744161, retrieved from STN Database accession no. 1966:18904 & JAKOBSEN, HANS JOERGEN ET AL: "Enamine chemistry. V . Introduction of the benzoyloxy group into some .beta.-oxoesters and .beta.-diketones. A new route to oxazoles", ARKIV FOER KEMI , 24(39), 519-30 CODEN: ARKEAD; ISSN: 0365-6128, 1965,
- DATABASE CAPLUS [Online] CHEMICAL ABSTRACTS SERVICE, COLUMBUS, OHIO, US; MORI, KIYOMI ET AL: "Hydrolyzable acrylic resin compositions and their use in marine antifouling agents", XP002744162, retrieved from STN Database accession no. 1993:149558 & JP H42 52209 A (NITTO KASEI CO., LTD., JAPAN) 8 September 1992 (1992-09-08)
- DATABASE CAPLUS [Online] CHEMICAL ABSTRACTS SERVICE, COLUMBUS, OHIO, US; OCHIAI, YUICHI ET AL: "Preparation of penam derivatives as antibiotics", XP002744163, retrieved from STN Database accession no. 1988:492644 & JP S62 228083 A (TOYAMA CHEMICAL CO., LTD., JAPAN) 6 October 1987 (1987-10-06)
- DATABASE CAPLUS [Online] CHEMICAL ABSTRACTS SERVICE, COLUMBUS, OHIO, US; WANG, XIAOYU ET AL: "Method for determining volatile and semivolatile secondary metabolites in fresh tobacco leaves", XP002744164, retrieved from STN Database accession no. 2012:1020875 & CN 102 565 233 A (ZHENGZHOU TOBACCO RESEARCH INSTITUTE OF CNTC, PEOP. REP. CHINA) 11 July 2012 (2012-07-11)
- HASHIMOTO, TAKUYA ET AL.: 'Phase-Transfer-Catalyzed Asymmetric Alkylation of ?-Benzoyloxy- ?-keto Esters: Stereoselective Construction of Congested 2,3-Dihydroxycarboxylic Acid Esters' CHEM. ASIAN J. vol. 5, no. 3, 10 December 2009, pages 562 - 570, XP055066391
- SCHEID, GÜNTHER ET AL.: 'A New Route to Protected Acyloins and Their Enzymatic Resolution with Lipases' EUR. J. ORG. CHEM. vol. 5, 2004, pages 1063 - 1074, XP055066388
- RUSSELL, GLEN A. ET AL.: 'Aliphatic Semidiones. V Radical Anions Derived from Vicinal Triketones' J. AM. CHEM. SOC. vol. 89, no. 25, 06 December 1967, pages 6623 - 6628, XP055167088
- LODAYA, JAYANT S. ET AL.: 'Direct a-Mesyloxylation of Ketones and ?-Dicarbonyl Compounds with [Hydroxy(mesyloxy)iodo]benzene' J. ORG. CHEM. vol. 53, no. 1, 1988, pages 210 - 212, XP055167089
- FIELD, LAMAR ET AL.: 'Oxidation Reaction with Lead(IV) Sulfonates' SULFUR LETTERS vol. 1, no. 6, October 1983, pages 181 - 189, XP008174762
- CORNFORTH, J. W. ET AL.: 'Synthesis of Oxazoles from Ethyl Acetoacetate. Ring-fission of Oxazole- 5-carboxylic Acids' JOURNAL OF THE CHEMICAL SOCIETY 01 January 1953, XP055167092
- YAMAMOTO, YUKIHARU ET AL.: 'PhI- and polymer-supported PhI-catalyzed oxidative conversion of ketones and alcohols to a-tosyloxyketones with m-chloroperbenzoic acid and p-toluenesulfonic acid' TETRAHEDRON vol. 63, no. 22, 18 March 2007, pages 4680 - 4687, XP022047398
- HU, JIANTAO ET AL.: 'A Novel One-Pot Method for a-Tosyloxylation of Ketones Using a Catalytic Amount of Ammonium Iodide' SYNTHESIS vol. 44, no. 8, 15 March 2012, pages 1226 - 1232, XP055167094
- PENZ, GOTTFRIED ET AL.: 'Synthese der (3-Oxo-2-tosyloxy-1-alkenyl)phosphonsauredi alkylester- <?PIHORS-JCR-BEGINcitations_suite ?> Synthone zur Gewinnung von (Hetarylmethyl)phosphonsaureestern und 2-substituierten ?-Oxophosphonsaureestern' CHEM. BER. vol. 118, no. 10, 1985, pages 4131 - 4143, XP008174763
- TRUKHIN, DMITRY V ET AL.: 'A Straightforward and Convenient Synthesis of Cbz-Protected 2-(1- Aminoalkyl)oxazole-5-carboxylates' SYNLETT vol. 13, 12 July 2005, pages 2072 - 2076, XP055066569
- TANAKA, A. ET AL.: "4-MeC6H4I-Mediated Efficient [alpha]-Tosyloxylation of Ketones with Oxone? and p-Toluenesulfonic Acid in Acetonitrile", SYNLETT, vol. 2009, no. 20, 2009, pages 3360-3364, XP55368093, ISSN: 0936-5214, DOI: 10.1055/s-0029-1218370
- TANAKA, A. ET AL.: "Iodoarene-Mediated [alpha]-Tosyloxylation of Ketones with MCPBA and p-Toluenesulfonic Acid", SYNLETT, vol. 2011, no. 13, 2011, pages 1853-1858, XP55368086, ISSN: 0936-5214, DOI: 10.1055/s-0030-1260948
- SUZUKI, Y. ET AL.: "Novel Preparation of Polymer-Supported Iodobenzene and Its Synthetic Utility as a Recyclable Reagent with m-Chloroperbenzoic Acid", SYNTHESIS, vol. 2010, no. 14, 2010, pages 2355-2360, XP55368071, ISSN: 0039-7881, DOI: 10.1055/s-0029-1218795

## Description

### Field of the Invention

The invention relates to a process for preparing an intermediate compound, which can be used to synthesize the known intermediate compound for preparing vitamin B₆, 4-methyloxazole-5-carboxylate.

### Background of the Invention

There have been many processes of preparing vitamin B₆, and Ullmann's Encyclopaedia of Industrial Chemistry, 5th version, 1996, vol. A27, Page 533-537 describes the most important ones. Normally, the process described in Kondratyeva, G.Y., Khim. Nauka Promst. 2, 666 (1957) is industrially used to synthesize vitamin B₆, in which the pyridine ring is obtained via Diels-Alder reaction between oxazole and maleic acid or derivative thereof. In this synthesis route, the specially preferred oxazole is 5-cyano-4-methyloxazole, which is usually prepared from the known intermediate compound 4-methyloxazole-5-carboxylate (see patent publications US 4,772,718 and EP 10697).

The patent publications US 3538110, IN 177708, US 4026901, US 2009143346 and so on disclose processes for preparing the above known intermediate 4-methyloxazole-5-carboxylate. US3538110 discloses a conventional process, which comprises reacting 2-chloro-acetoacetate with formamide (EACA) to obtain ethyl 4-methyloxazole-5-formate (OXE), and additional formic acid and ammonium chloride.

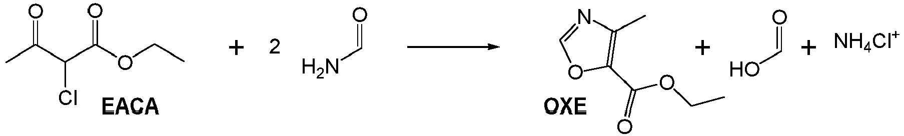

However, the process is limited due to low yield, only 63%. In addition, the process has a drawback of high consumption of expensive formamide. It needs consume 2 stoichiometric equivalents of formamide, and increase to 3 equivalents in the practical operation.

WO2011108696 discloses a process for producing an α-acyloxycarbonyl compound by reacting a carboxylic acid and a carbonyl compound selected from the group consisting of ketones, aldehydes, and esters with a hydroperoxide as an oxidizer and an iodide salt as a catalyst precursor. However, the α-acyloxycarbonyl compound cannot be used to produce OXE.

Takuya Hashimoto *et al.* (Takuya Hashimoto et al., Chem. Asian J. 2010, 5, 562-570) and Günther Scheid *et al.* (Günther Scheid et al. , Eur. J. Org. Chem. 2004, 1063-1074) discloses a two-step process for producing tert-butyl acetoxyacetoacetate by bromination of tert-butyl acetoacetate with NBS to give a bromo derivative, followed by nucleophilic substitution with sodium acetate in DMF. However, the yield of the process is only 69%.

Therefore, a new process for preparing the known intermediate compound 4-methyloxazole-5-carboxylate is still in need.

### Summary of the Invention

The invention provides a process for preparing an intermediate compound, which can be used to synthesize the known intermediate compound for preparing vitamin B₆, 4-methyloxazole-5-carboxylate. The process of using the intermediate compound to prepare 4-methyloxazole-5-carboxylate is simple, uses cheap raw material, and does not use chlorine- and phosphorus-containing compounds which are harmful to environment, so the process is simple, economic and environmentally friendly.

The process according to the invention provides an intermediate compound of formula (I):

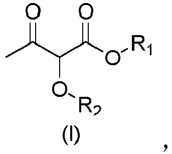

Wherein:
R₁ is H or C₁-C₁₀ hydrocarbyl; and
R₂ is C₁-C₄ acyl.

The process for preparing the compound of formula (I) comprises the step (a) reacting a compound of formula (II) with a compound of formula (III) in the presence of an optionally substituted iodobenzene catalyst, 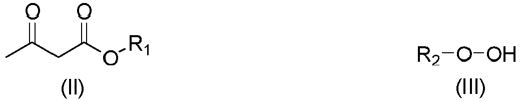 wherein R₁ and R₂ are defined as above.

### Detailed description of the Invention

"C₁-C₁₀ hydrocarbyl" refers to linear or branched chain, saturated or unsaturated, cyclic or non-cyclic hydrocarbyl comprising 1-10 carbon atoms, including alkyl, alkenyl, alkynyl and aryl. Preferably, the "C₁-C₁₀ hydrocarbyl" is C₁-C₄ hydrocarbyl, including alkyl, alkenyl and alkynyl, for example but not limited to methyl, ethyl, propyl, isopropyl, cyclopropyl, butyl, isobutyl, tert-butyl, methyl cyclopropyl, cyclobutyl, vinyl, propenyl, butenyl, ethynyl, propynyl and butynyl group. More preferably, the "C₁-C₁₀ hydrocarbyl" is methyl or ethyl.

"C₁-C₁₀ acyl" refers to a group with the structure of RC(O)-, wherein R is H or linear or branched chain, saturated or unsaturated, cyclic or non-cyclic hydrocarbyl comprising 1-9 carbon atoms, including alkyl, alkenyl, alkynyl and aryl. Preferably, the "C₁-C₁₀ acyl" is C₁-C₄ acyl, for example but not limited to formyl, acetyl, propionyl, butyryl and isobutyryl. More preferably, the "C₁-C₁₀ acyl" is formyl.

"C₁-C₁₀ sulfonyl" refers to a group with the structure of RS(O)₂-, wherein R is C₁-C₁₀ hydrocarbyl, for example, aryl such as phenyl; alkaryl such as C₁-C₁₀ alkylaryl; and aralkyl such as C₁-C₁₀ aralkyl. Preferably, "C₁-C₁₀ sulfonyl" is phenylsulfonyl, p-toluenesulfonyl or methyl sulfonyl.

The process according to the invention provides a compound of formula (I):

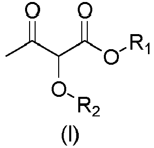

Wherein:
R₁ is H or C₁-C₁₀ hydrocarbyl; and
R₂ is C₁-C₄ acyl.

In an embodiment, R₁ is C₁-C₄ hydrocarbyl, and R₂ is C₁-C₄ acyl. In another embodiment, R₁ is C₁-C₄ hydrocarbyl, and R₂ is formyl, acetyl, propionyl, butyryl or isobutyryl. In a preferred embodiment, R₁ is methyl or ethyl, and R₂ is formyl.

In another embodiment, the compound of formula (I) is selected from a group consisting of methyl 2-formyloxy-3-oxy-butyrate, and ethyl 2-formyloxy-3-oxy-butyrate (FOXE). In a preferred embodiment, the compound of formula (I) is ethyl 2-formyloxy-3 -oxy-butyrate.

The invention provides a process for preparing the compound of formula (I), comprising the following step:
(a) reacting a compound of formula (II) with a compound of formula (III) in the presence of a catalyst of substituted or unsubstituted iodobenzene, 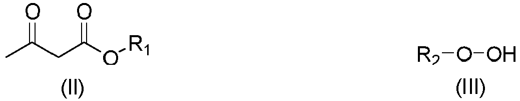
Wherein: R₁ and R₂ are defined as above.

The examples of the compound of formula (II) include methyl acetoacetate, ethyl acetoacetate, propyl acetoacetate, butyl acetoacetate, vinyl acetoacetate, propenyl acetoacetate or ethynyl acetoacetate. The examples of the compound of formula (III) include peroxyformic acid, peroxyacetic acid, or peroxypropionic acid. In an embodiment, the compound of formula (II) is ethyl acetoacetate, and the compound of formula (III) is peroxyformic acid.

In the step (a), the catalyst is substituted or unsubstituted iodobenzene.

The most preferred catalyst is unsubstituted iodobenzene.

In the step (a), the molar ratio of the catalyst and the compound of formula (II) is 0.0001-2 : 1, 0.005-0.5 : 1, preferably 0.01-0.1 : 1, more preferably 0.05 : 1.

In the step (a), the reaction temperature for the reaction between the compound of formula (II) and the compound of formula (III) is in the range of -30°C to 150°C, preferably -10°C to 100°C, more preferably 0°C to 60°C.

Optionally, the reaction of step (a) may be carried out in a solvent. The solvent may be any available solvent known in the art, the examples of which include but are not limited to nitrile such as acetonitrile, dichloromethane, benzene, organic acids including but not limited to formic acid and acetic acid, etc., and the mixture thereof.

Optionally, the reaction of step (a) may be carried out with the protection of nitrogen gas. Gas chromatography may be used to monitor the reaction between the compound of formula (II) and the compound of formula (III). After the reaction stops, the solid impurities that may be produced are removed by filtration and then the catalyst and the solvent are recovered by fractionation to give a liquid crude product of the compound of formula (I). The yield of the compound of formula (I) may be calculated by quantitative gas chromatography.

In the process, the compound of formula(II) is available commercially while the compound of formula (III) is preferably obtained by the process known in the art, such as the process disclosed in the DE 2262970 A1, DE 2519293 A1, JP 2006219379 A, EP 641777 A1, *etc..*

Preferably, the compound of formula (III) is obtained by the following step (b):
(b) oxidizing a compound of formula (IV) with an oxidant to give the compound of formula(III).

R₂-OH (IV)

wherein R₂ is defined as above.

The compound of formula (IV) is an organic acid, such as formic acid, acetic acid, propionic acid.

In the step (b), the oxidant may be any available oxidant know in the art, including but not limited to m-chloroperoxybenzoic acid, hydrogen peroxide, sodium percarbonate, urea hydrogen peroxide, potassium peroxysulfate and oxone, *etc..* The preferred oxidant is m-chloroperoxybenzoic acid and hydrogen peroxide.

In the step (b), the reaction temperature of the oxidation may be the same as that in the step (a), in the range of -30°C to 150°C, preferably -10°C to 100°C, and more preferably 0°C to 60°C.

Optionally, the reaction of the step (b) may be carried out in a solvent which may be the same with or different from that/those used in the step (a). It is understandable for one ordinary skilled in the art that, when the compound of formula (IV) is organic acid, it may be used as a substrate or a solvent per se.

In the process, the step (b) and the step (a) may be carried out in the same or different reactors. In an embodiment, the step (b) and the step (a) are carried out in different reactors. The mixture from the reaction of the step (b) is directly used into the step (a). In another embodiment, the step (b) and the step (a) are carried out in the same reactor.

In the embodiment where the step (b) and the step (a) are carried out in the same reactor, the invention provides a new "one-pot" process for preparing the compound of formula (I), comprising reacting the compound of formula (II) with the compound of formula (IV) in the same reactor in the presence of the oxidant and the catalyst to prepare the compound of formula (I).

In the "one-pot" process, the compound of formula (II) and the compound of formula (IV) can be simultaneously added into the reactor; or the compound of formula (IV) and the catalyst are added into the reactor firstly and then the oxidant and /or the compound of formula (II) are added into the reaction to prepare the compound of formula (I).

In the "one-pot" process, the reaction temperature is in the range of -30°C to 150°C, more preferably -10°C to 100°C, and more preferably 0°C to 60°C.

Optionally, the "one-pot" process may be carried out in a solvent. The solvent may be any available solvent known in the art, including but not limited to nitrile such as acetonitrile, dichloromethane, benzene, and organic acid including but not limited to formic acid and acetic acid *etc.,* or mixture thereof. It is understandable for one ordinary skilled in the art that, when the compound of formula (IV) is organic acid, it may be used as a substrate or a solvent per se. Optionally, the "one-pot" process may be carried out with the protection of nitrogen gas. Gas chromatography may be used to monitor the reaction. After the reaction stops, the solid impurities that may be produced are removed by filtration and then the catalyst and the solvent are recovered by fractionation to give a liquid crude product of the compound of formula (I). The yield of the compound of formula (I) may be calculated by quantitative gas chromatography.

The process provided by the invention is easy to operate, and the compound of formula (I) can be obtained via the one-step reaction from the compound of formula (II) or via the "one-pot" process from the compound of formula (IV). The process uses the raw material which is simple and easy to obtain, does not produce any chlorine and sulfur wastes which are harmful to environment, and can recover the catalyst and solvent easily. Thereby, the process is simple, economic and environmental friendly.

### Examples

The present invention is illustrated further by the following examples.

### Example 1: Preparation of ethyl 2-formyloxy-3-oxy-butyrate (FOXE) using peroxyformic acid

3.7 g ethyl acetoacetate (28.5 mL), 1.8g peroxyformic acid and 0.4 g iodobenzene(1.9 mmol) were added into a 1000 mL flask equipped with thermometer for reaction at 25-30°C for 30-40 mins under nitrogen gas. After gas chromatography tracking determined that the reaction had finished, the yield was calculated as 90% by quantitative gas chromatography. **¹H NMR**(CDCl₃): 1.29(t, 3H), 2.13(s, 3H), 4.21(q, 2H), 5.66(s, H), 8.04(s, H)

### Example 2: Preparation of FOXE using formic acid

100 mL formic acid and 7.3 g sodium percarbonate (2Na₂CO₃•3H₂O₂, 23 mmol) were added into a 250 mL round-bottomed flask equipped with magnetic stirrer and thermometer for reaction at 20-30°C. 30 mins later, 7.0 g ethyl acetoacetate (54 mL) and 0.55 g iodobenzene (2.7 mmol) were added into the reaction mixture in one portion for reaction at 20-30°C. 2 hours later, gas chromatography indicated that ethyl acetoacetate had been used up. The residual formic acid and iodobenzene were recovered by distillation under vacuum, and then the obtained ethyl 2-formyloxy-3-oxy-butyrate was recovered by distillation under vacuum. The yield was 85%, calculated by quantitative gas chromatography.

**¹H NMR**(CDCl₃): 1.29(t, 3H), 2.13(s, 3H), 4.21(q, 2H), 5.66(s, H), 8.04(s, H)

### Example 3: Preparation of ethyl 2-methylsulphonyloxy-3-oxy-butyrate (not according to the invention)

7.5 g ethyl acetoacetate(58 mL), 0.58 g iodobenzene (2.8 mmol), 11.1 g methylsulphonic acid (116 mmol) and 100 mL acetonitrile were added into a 250 mL round-bottomed flask equipped with magnetic stirrer and thermometer, and 18.6 g m-chloroperoxybenzoic acid (75.4 mmol) was added into the mixture in several portions. Afterwards, the mixture was heated to 60°C and kept for 4 hours. After gas chromatography indicated that ethyl acetoacetate had been used up, the produced solid was removed by filtration and then the solvent was removed by evaporation to give ethyl 2-methylsulphonyloxy-3-oxy-butyrate (yield 62.15%).

**¹H NMR**(CDCl₃): 1.29(t, 3H), 2.13(s, 3H), 3.16(q, 2H), 4.21(q, 2H), 6.49(s, H)

### Example 4: Preparation of vinyl 2-formyloxy-3-oxy-butyrate

100 mL formic acid and 15.9 g m-chloroperoxybenzoic acid (65 mmol) were added into a 250 mL round-bottomed flask equipped with magnetic stirrer and thermometer for reaction at 20-30°C. 30 mins later, 6.9 g vinyl acetoacetate (54 mL) and 0.55 g iodobenzene (2.7 mmol) were added into the reaction mixture in one portion for reaction at 20-30°C. 2 hours later, gas chromatography indicated that vinyl acetoacetate had been used up. The residual formic acid and iodobenzene were recovered by distillation under vacuum, and then the obtained vinyl 2-formyloxy-3-oxy-butyrate was recovered by distillation under vacuum. The yield was about 82%, calculated by quantitative gas chromatography.

**¹H NMR**(CDCl₃): 2.13(s, 3H), 4.55(q, 1H), 4.85(q, 1H), 4.21(q, 2H), 5.66(s, H), 7.25(dd, 1H), 8.04(s, 1H)

### Example 5: Preparation of 2-formyloxy-3-oxy-butyric acid

100 mL formic acid and 15.9 g m-chloroperoxybenzoic acid (65 mmol) were added into a 250 mL round-bottomed flask equipped with magnetic stirrer and thermometer for reaction at 20-30°C. 30 mins later, 5.5 g acetoacetate (54 mmol) and 0.55 g iodobenzene(2.7 mmol) were added into the reaction mixture in one portion for reaction at 20-30°C. 2 hours later, gas chromatography indicated that acetoacetate had been used up. The residual formic acid and iodobenzene were recovered by distillation under vacuum, and then the obtained 2-formyloxy-3-oxy-butyric acid was recovered by distillation under vacuum. The yield was about 82%, calculated by quantitative gas chromatography.

**¹H NMR**(CDCl₃): 2.13(s, 3H), 5.70(s, 1H), 8.04(s, 1H), 11.0(s, 1H)

### Example 6: Preparation of ethyl 2-hydroxyl-3-oxy-butyrate (not according to the invention)

100 mL formic acid and 7.3 g sodium percarbonate(2Na₂CO₃•3H₂O₂, 23 mmol) were added into a 250 mL round-bottomed flask equipped with magnetic stirrer and thermometer for reaction at 20-30°C. 30 mins later, 7.0 g ethyl acetoacetate (54 mL) and 0.55 g iodobenzene(2.7 mmol) were added into the reaction mixture in one portion for reaction at 20-30°C. 2 hours later, gas chromatography indicated that ethyl acetoacetate had been used up, and then the reaction was kept for additional 1 hour. The residual formic acid and iodobenzene were recovered by distillation under vacuum and the obtained ethyl 2-hydroxyl-3-oxy-butyrate was recovered by distillation under vacuum. The yield was 85%, calculated by quantitative gas chromatography.

**¹H NMR**(CDCl₃): 1.29(t, 3H), 2.13(s, 3H), 3.65(s, 1H), 4.21(q, 2H), 4.92(s, 1H)

### Example 7: Preparation of ethyl 4-methyloxazole-5-formate (OXE)

150.2 g formamide (98%, 3.267 mol, 1.60 eq.) and 13.4 g sulfuric acid (98%, 0.135 mol, 0.07 eq.) were added into a 500 mL stirred reactor equipped with a 30 mm×30 cm Kerapak column. The mixture was heated to 120°C under the pressure of 65 mbar. Later 400.2 g FOXE crude product (88.6%, 2.036 mol, 1.00 eq.) was added in 5 hours. HOOCH/water was distilled out from the reaction mixture during the addition of FOXE. After the addition of FOXE finished, HOOCH/water was continued to be distilled out until the reaction was completed. During the whole reaction, 100.8 g HCOOH/mixture (HCOOH 56.7% and water 37.7%) was distilled out, and 407.0 g OXE crude product and deposited ammonium sulphate were obtained. 400.5 g OXE crude product and deposited ammonium sulphate were filtrated to give 6.0 g filter cake and 391.5 g OXE crude product filtrate (OXE 68.7%). The total yield was 91.9%.

The filter cake was washed by 11.0 g FOXE crude product twice to give 4.8 g filter cake without OXE and 24.2 g filtrate (FOXE 76.9% and OXE 2.0%). The total yield of OXE was 92.5%.

## Claims

1. A process for preparing a compound of formula (I), 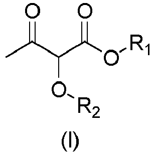 comprising the step:
(a) reacting a compound of formula (II) with a compound of formula (III) in the presence of a catalyst of substituted or unsubstituted iodobenzene, 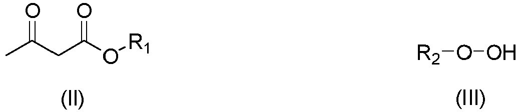 wherein:
R₁ is H or C₁-C₁₀ hydrocarbyl; and
R₂ is C₁-C₄ acyl.

2. The process of claim 1, wherein the compound of formula (II) is methyl acetoacetate, ethyl acetoacetate, propyl acetoacetate, butyl acetoacetate, vinyl acetoacetate, propenyl acetoacetate or ethynyl acetoacetate.

3. The process of claim 1, wherein the compound of formula(III) is peroxyformic acid, peroxyacetic acid, or peroxypropionic acid.

## Patentansprüche

1. Verfahren zur Herstellung einer Verbindung der Formel (I) 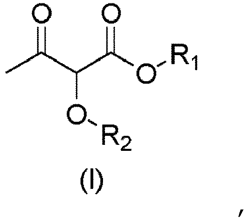 welches den folgenden Schritt umfasst:
(a) die Umsetzung einer Verbindung der Formel (II) mit einer Verbindung der Formel (III) in Gegenwart eines Katalysators von substituiertem oder unsubstituiertem Iodbenzol 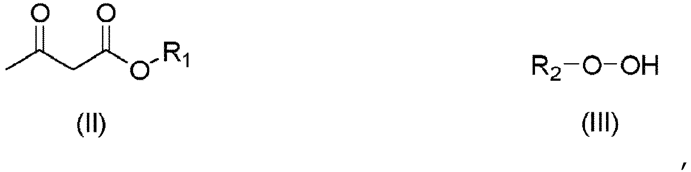 wobei:
R₁ für H oder C₁-C₁₀-Hydrocarbyl steht und
R₂ für C₁-C₄-Acyl steht.

2. Verfahren nach Anspruch 1, wobei es sich bei der Verbindung der Formel (II) um Acetoessigsäuremethylester, Acetoessigsäureethylester, Acetoessigsäurepropylester, Acetoessigsäurebutylester, Acetoessigsäurevinylester, Acetoessigsäurepropenylester oder Acetoessigsäureethinylester handelt.

3. Verfahren nach Anspruch 1, wobei es sich bei der Verbindung der Formel (III) um Peroxyameisensäure, Peroxyessigsäure oder Peroxypropionsäure handelt.

## Revendications

1. Procédé pour la préparation d'un composé de formule (I), 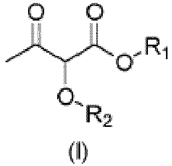 comprenant l'étape de :
(a) mise en réaction d'un composé de formule (II) avec un composé de formule (III) en la présence d'un catalyseur d'iodobenzène substitué ou non substitué, 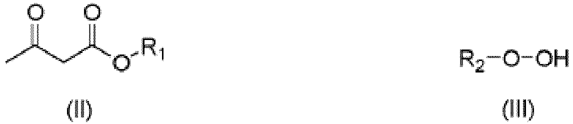
R₁ étant H ou C₁₋₁₀-hydrocarbyle ; et
R₂ étant C₁₋₄-acyle.

2. Procédé selon la revendication 1, le composé de formule (II) étant l'acétoacétate de méthyle, l'acétoacétate d'éthyle, l'acétoacétate de propyle, l'acétoacétate de butyle, l'acétoacétate de vinyle, l'acétoacétate de propényle ou l'acétoacétate d'éthynyle.

3. Procédé selon la revendication 1, le composé de formule (III) étant l'acide peroxyformique, l'acide peroxyacétique, ou l'acide peroxypropionique.
